# EUROPEAN PATENT APPLICATION

(11) **EP 4 292 595 A1**
(43) Date of publication of application: **20.12.2023**
(21) Application number: 22752843.7
(22) Date of filing: 10.02.2022
(51) Int. Cl.: A61K 31/497, A61P 35/00, C12Q 1/6827

(54) **APPLICATION OF GILTERITINIB TO VARIOUS MUTANTS**

(30) Priority: 12.02.2021 JP 2021020640
(71) Applicant: Japanese Foundation For Cancer Research, Tokyo 135-8550 (JP)
(72) Inventor: KATAYAMA Ryohei, Tokyo 135-8550 (JP)
(74) Representative: Schön, Christoph
(86) International application number: PCT/JP2022/005499
(87) International publication number: WO 2022/173021

(57) **Abstract**

Tumors having acquired resistance to ALK-TKIs were analyzed by use of various kinase inhibitors. As a result, it was found that gilteritinib, which is a therapeutic drug for FLT3 gene mutation-positive acute myeloid leukemia, has an effect on the tumors. Gilteritinib directly inhibits the kinase activity of ALK to have an effect on an ALK fusion gene mutation-positive tumor having a plurality of compound mutations for which effective therapeutic drugs have been not present. ALK-TKI-resistant cancer developed via a fusion gene such as NTRK, ROS1 and LTK, and via AXL can be effectively overcome by gilteritinib alone, and the resistance mediated through ALK-TKI resistance mechanism via a bypass pathway such as KRAS, BRAF and EGFR, can be overcome by combination therapy.

## Description

### Technical Field

The present invention relates to a drug effective for an ALK fusion gene-positive tumor having acquired resistances to a plurality of drugs and application range thereof.

### Background Art

As cancer medication, molecular target drugs have been developed in response to various genetic mutations and the therapeutic effects of the drugs have been improved. The molecular target drugs are delivered to driver oncogenes as targets and control their functions, thereby treating cancers. Because of this, the side effects of most molecular target drugs are relatively milder than those of conventional cytotoxic anticancer agents. However, even if a drug works and cancer shrinks, drug resistance develops with time and the drug becomes ineffective. Such drug resistance causes problems.

Lung cancer is one of the cancers having a large number of patients. The number of individuals developing lung cancer per year is about 125,000 in Japan, which is the 3rd highest among all cancers. From a global point of view, the number of individuals having lung cancer is large and 2,000,000 or more individuals are newly affected with lung cancer per year. The types of lung cancer are roughly divided into two: small-cell lung cancer (SCLC) and non-small-cell lung cancer (NSCLC). The majority of the patients are affected with non-small cell lung cancer.

It has been reported that non-small cell lung cancer has many driver gene mutations; and that not only genetic mutations in EGFR and K-RAS but also fusion genes such as ALK, ROS1 and NTRK are frequently found as typical gene abnormalities. It has been reported that the ALK fusion genes are found in a ratio of 3 to 5% in the non-small cell lung cancer patients. The ratio is the highest in lung cancer caused by fusion gene mutations. The ALK fusion gene refers to an oncogene generated by the fusion of a receptor tyrosine kinase (ALK) gene and a gene encoding a protein, such as EML4, having a multimer-forming domain. The ALK fusion protein forms a multimer depending on the function of a fusion partner protein for ALK and acquires constant kinase activity. This is a possible cause of cancer.

It has been clinically shown that molecular target drugs that specifically inhibit ALK tyrosine kinase are effective for ALK-positive lung cancer. Up to the present, five ALK tyrosine kinase inhibitors (ALK-TKIs) including crizotinib, which was a first developed ALK-TKI, have been clinically applied. However, cancer cells acquire drug resistance after treatment as mentioned above, resulting in the recurrence of cancer. Recurrence is a matter of concern.

Alectinib, which is shown to have progression-free survival (PFS) of about three times as long as that of crizotinib, has been used as a primary therapeutic drug for ALK-positive lung cancer. However, the problem is that drug resistance develops in most cases. Mutations in the kinase domain, such as G1202R and I1171N/S/T, are detected as alectinib-resistant mutations. For about half of these mutations, lorlatinib, developed as a third-generation ALK-TKI, has been reported to be effective. However, it has been reported that compound mutations, which occur in the ALK kinase domain, acquire resistance even against lorlatinib. Some of the compound mutations become again sensitive to crizotinib and alectinib but other compound mutations such as G1202R and L1196M (hereinafter referred to as G1202R+L1196M) are resistant against all ALK-TKIs.

### Citation List

### Non Patent Literatures

Non Patent Literature 1: Okada K, et al., 2019, EBioMedine Vol. 41, pp. 105-119
Non Patent Literature 2: Shaw AT, et al., 2016, N Engl J Med, Vol. 374, pp.54-61
Non Patent Literature 3: Yoda S, et al. 2018, Cancer Discov Vol. 8, pp. 714-729
Non Patent Literature 4: Recondo G, et al. 2020, Clin Cancer Res Vol. 26, pp. 242-255
Non Patent Literature 5: Taniguchi H, et al. 2019, Nat Commun Vol. 10, 259
[Non Patent Literature 6: Doebele RC, et al., 2012, Clin Cancer Res Vol. 18, pp. 1472-1482
Non Patent Literature 7: Hrustanovic G, et al., 2015, Nat Med Vol. 21, pp. 1038-1047
Non Patent Literature 8: Maynard A, et al., 2020, Cell Vol.182, pp.1232-1251 e1222

### Summary of Invention

### Technical Problem

As mentioned above, molecular target drugs exert extremely high effects but the development of resistance creates problems. An object of the present invention is to provide a medicine having an effect on ALK-positive tumors having acquired resistances against all drugs, and, in particular, to find a drug exerting an effect on compound mutations, such as I1171N+F1174I and I1171N+L1198H. Another object of the invention is to find a molecule that can serve as a target for the medicine, in other words, an effective genetic mutation based on the similarity of the structures to which a drug binds, thereby expanding the application range of drugs to widen the selection range of therapies. Particularly, the application range of gilteritinib, which was found to overcome the aforementioned compound mutations, was investigated.

### Solution to Problem

The present invention relates to the application of the following drug.
(1) A pharmaceutical composition for treating an ALK fusion gene-positive tumor, containing gilteritinib as an active ingredient.
(2) The pharmaceutical composition for treating an ALK fusion gene-positive tumor according to (1), wherein the ALK fusion gene-positive tumor is a tumor having a wild type ALK or acquired resistance to a first-generation, a second-generation of ALK-TKIs or lorlatinib.
(3) The pharmaceutical composition for treating an ALK fusion gene-positive tumor according to (1) or (2), wherein the ALK fusion gene-positive tumor is compound mutations with I1171N, I1171S, V1180L, L1196M or C1156Y.
(4) The pharmaceutical composition for treating an ALK fusion gene-positive tumor according to any one of (1) to (3), wherein the ALK fusion gene-positive tumor is an ALK fusion gene having at least two compound mutations including mutations: I1171N/S/T, F1174I/L, L1196M, L1198F/H, G1202R, D1203N, F1245V, L1256F and G1269A.
(5) The pharmaceutical composition for treating an ALK fusion gene-positive tumor according to any one of (1) to (3), wherein the ALK fusion gene-positive tumor has at least one ALK mutation of T1151K, C1159Y, I1171N, I1171T, I1171S, F1174I, F1174V, V1180L, L1196M, L1196Q, L1198F, D1203N, F1245V, L1256F or G1269A.
(6) The pharmaceutical composition for treating an ALK fusion gene-positive tumor according to any one of (1) to (5), wherein the ALK fusion gene-positive tumor is a non-small cell lung cancer.
(7) A pharmaceutical composition according to (6) for treating non-small cell lung cancer, for use as a primary therapy or a secondary therapy.
(8) A composition for treating a tumor caused by activation of AXL, an NTRK fusion gene, an LTK fusion gene and/or a ROS1 fusion gene, containing gilteritinib as an active ingredient.
(9) A method for examining whether gilteritinib exerts an effect on a patient with an ALK fusion-gene positive tumor, including: examining whether a kinase domain of ALK is a wild type or has at least one ALK mutation of T1151K, C1159Y, I1171N, I1171T, I1171S, F1174I, F1174V, V1180L, L1196M, L1196Q, L1198F, D1203N, F1245V, L1256F or G1269A; and determining that gilteritinib is effective when any one of the above present.
(10) A method for examining whether gilteritinib exerts an effect on a patient with an ALK fusion-gene positive tumor, including examining whether G1202R or D1203 mutation is present; determining that gilteritinib does not exert an effect if G1202R is present alone, or determining that the effect of gilteritinib may be slightly attenuated if a compound mutant of D1203 mutation or G1202R mutation and another mutation is present.
(11) A therapeutic method including: examining a cause of a disease in a subject; and administering a pharmaceutical composition containing gilteritinib as an active ingredient if the cause is any one of an ALK fusion gene positive, activation of AXL including Gas6 expression induction, NTRK1 fusion gene, LTK fusion gene and/or ROS1 fusion gene mutations.
(12) The therapeutic method according to (11),
   wherein the disease in a subject is an ALK fusion gene-positive tumor having a wild-type ALK or acquired resistance to first-generation, second-generation of ALK-TKIs, or lorlatinib.
(13) A therapeutic method for a tumor having developed resistance to ALK-TKI, including: examining a mechanism of acquisition of the resistance; and administering gilteritinib alone if the mechanism of acquisition of the resistance involves activation of AXL; administering gilteritinib and a KRAS inhibitor in combination if the resistance is acquired via KRAS bypass pathway, administering gilteritinib, a BRAF inhibitor and/or a MEK inhibitor in combination if the mechanism of acquisition of the resistance is acquired via BRAF bypass pathway, or administering gilteritinib and an EGFR inhibitor in combination if the resistance is acquired via EGFR bypass pathway.

### Brief Description of Drawings

[Figure 1A] The figure shows that gilteritinib suppresses phosphorylation of ALK and ALK compound mutations, based on western blotting.
[Figure 1B] The figure shows the inhibitory effects of alectinib, lorlatinib and gilteritinib on phosphorylation of ALK and ALK compound mutations and activation of the signaling pathway downstream thereof, based on western blotting analysis.
[Figure 1C] The figure shows the inhibitory effects of alectinib, lorlatinib and gilteritinib on phosphorylation of ALK and ALK compound mutations and activation of the signaling pathway downstream thereof, based on western blotting analysis.
[Figure 2A] The figure shows that the inhibitory activity of gilteritinib on ALK kinase decreases depending on the ATP concentration, and that is, gilteritinib directly inhibits ALK competitively with ATP.
[Figure 2B] The figure shows the results of phosphoproteome analysis showing a change in phosphorylation of peptides treated with gilteritinib.
[Figure 3A] The figure shows the cell proliferation inhibitory effects (IC₅₀) of gilteritinib and ALK-TKIs on ALK gene mutation-positive non-small cell lung cancer cell lines and non-Hodgkin's lymphoma cell lines.
[Figure 3B] The figure shows autophosphorylation of ALK in ALK gene mutation-positive cancer cell lines, based on western blotting analysis.
[Figure 3C] The figure shows that gilteritinib induces apoptosis.
[Figure 4A] The figure shows the effects of treatment with gilteritinib on the activities of a receptor tyrosine kinase in non-small cell lung cancer cells and normal human fibroblasts.
[Figure 4B] The figure shows the effect of gilteritinib evaluated by xenograft mouse models.
[Figure 5A] The figure shows the viability rates (IC₅₀) of Ba/F3 cells expressing ALK mutants having single mutations observed in tumors having acquired resistances against first-generation and second-generation ALK-TKIs in the cells, and analyzed by treating the cells with lorlatinib, alectinib and gilteritinib.
[Figure 5B] The figure shows the analysis of viability rates (IC₅₀) of Ba/F3 cells having EML4-ALK mutants expressed therein and treated with gilteritinib and TKIs known to be effective for the ALK mutants.
[Figure 6] The figure shows autophosphorylation of ALK of Ba/F3 cells having single ALK mutants observed in tumors having acquired resistances against first-generation and second-generation ALK-TKIs, treated with gilteritinib and analyzed by western blotting.
[Figure 7A] The figure shows suppression of activation of the downstream signaling pathway in MCC-003 cells expressing EML4-ALK I1171N, treated with gilteritinib, and analyzed by western blotting.
[Figure 7B] The figure shows the analysis of apoptosis induction of MCC-003 cells, treated with gilteritinib or lorlatinib.
[Figure 8A] The figure shows the effect of gilteritinib evaluated by transplanting MCC-003 into mice.
[Figure 8B] The figure shows the in-vivo effect of gilteritinib on MCC-003 cells transplanted into mice based on autophosphorylation of ALK and activation of the signaling pathway downstream thereof.
[Figure 9A] The figure schematically shows the timing of the treatments with ALK-TKIs and ALK mutations detected by biopsy in a non-small cell lung cancer patient JFCR-049.
[Figure 9B] The figure shows the effects of gilteritinib on the viability rates of Ba/F3 cells in which ALK mutants detected in patient JFCR-049 were expressed.
[Figure 9C] The figure shows the viability rates (IC₅₀) of Ba/F3 cells having compound mutations expressed therein and treated with alectinib, lorlatinib and gilteritinib.
[Figure 9D] The figure shows the autophosphorylation of ALK in Ba/F3 cells having compound mutations expressed therein and treated with gilteritinib.
[Figure 10] The figure shows the effects of alectinib, lorlatinib and gilteritinib analyzed by use of xenograft models using JFCR-028-3 cells having EML4-ALK I1171N+F1174I expressed therein. A cytoreductive effect was observed by treating with alectinib or lorlatinib, followed by gilteritinib.
[Figure 11A] The figure schematically shows the timing of treatment with ALK-TKIs and ALK mutations (detected by biopsy) in non-small cell lung cancer patients, JFCR-134 and JFCR-016.
[Figure 11B] The figure shows the effect of gilteritinib on the viability rate of Ba/F3 cells having an ALK mutant having G1202R or D1203N mutation expressed therein.
[Figure 11C] The figure shows the inhibitory activity of ALK-TKIs and gilteritinib on MR347 cells (EML4-ALK-D1203N+L1196M) established from a non-small cell lung cancer patient resistant against the ALK-TKIs.
[Figure 11D] The figure shows IC₅₀ values of alectinib, lorlatinib and gilteritinib to Ba/F3 cells having compound mutations G1202R+L1196M, D1203N+F1245V, and D1203N+L1196M expressed therein.
[Figure 11E] The figure shows ALK autophosphorylation when Ba/F3 cells having compound mutations G1202R+L1196M, D1203N+F1245V, D1203N+L1196M expressed therein were treated with gilteritinib.
[Figure 12A] The figure shows the activations of AXL and the signaling pathway downstream of AXL by alectinib and gilteritinib in H3122 cells having AXL overexpressed therein, based on western blotting analysis.
[Figure 12B] The figure shows IC₅₀ values of alectinib and gilteritinib to H3122 cells having AXL overexpressed therein.
[Figure 13A] The figure shows the effect of gilteritinib analyzed by use of H3122 cell xenograft models having AXL overexpressed therein.
[Figure 13B] The figure shows the effect of alectinib analyzed by H3122 cell xenograft models.
[Figure 14A] The figure shows the effects of gilteritinib, trametinib and AMG510 on the KRAS signaling pathway by using MCC-003 cells having KRAS G12C expressed therein, based on western blotting analysis.
[Figure 14B] The figure shows the IC₅₀ value of gilteritinib to MCC-003 cells having KRAS G12C expressed therein.
[Figure 14C] The figure shows the effects of gilteritinib and AMG510 on the KRAS signaling pathway by using JFCR-028-3 cells having KRAS G12C expressed therein, based on western blotting analysis.
[Figure 14D] The figure shows IC₅₀ value of gilteritinib to JFCR-028-3 cells having KRAS G12C expressed therein.
[Figure 15A] The figure shows the effect of gilteritinib examined in a xenograft model using MCC-003 cells having KRAS G12C expressed therein.
[Figure 15B] The figure shows the effect of gilteritinib examined in xenograft models using JFCR-028-3 cells having KRAS G12C expressed therein.
[Figure 16A] The figure shows the viability rates of JFCR-098 cells established from a patient having acquired ALK-TKI resistance by the EGFR signaling pathway and treated with gilteritinib, alectinib and afatinib.
[Figure 16B] The figure shows the suppression of activations of EGFR and the signaling pathway downstream thereof by gilteritinib and alectinib, based on western blotting analysis.
[Figure 17A] The figure shows the effects of ALK-TKIs on viability rate analyzed by using TPM3-NTRK1 fusion gene-positive colorectal cancer cells KM12.
[Figure 17B] The figure shows the effect of ALK-TKIs on viability rate analyzed by use of Ba/F3 having a TPM3-NTRK1 fusion gene expressed therein.
[Figure 17C] The figure shows the effect of gilteritinib on NTRK1 and the signaling pathway downstream thereof, analyzed by using KM12 cells.
[Figure 17D] The figure shows the effect of entrectinib and gilteritinib on apoptosis analyzed by using KM12 cells.
[Figure 17E] The figure shows the effect of entrectinib and gilteritinib in a xenograft model having KM12 cells transplanted therein.
[Figure 17F] The figure shows the effect of entrectinib and gilteritinib on NTRK1 and the signaling pathway downstream thereof in a transplanted tumor based on western blotting analysis.
[Figure 18A] The figure shows the effect of gilteritinib on viability rate analyzed by using Ba/F3 cells in which TPM3-NTRK1 having G667C mutation was expressed.
[Figure 18B] The figure shows IC₅₀ values of gilteritinib and entrectinib to G595R mutation and G667C mutation of NTRK1 fusion gene.
[Figure 18C] The figure shows the effects of gilteritinib, entrectinib and lorlatinib on the viability rate analyzed by using Ba/F3 cells in which TPM3-NTRK1 having the G595R mutation was expressed.
[Figure 18D] The figure shows the suppression effect of gilteritinib on NTRK1 activity in the G667C mutation and G595R mutation of the NTRK fusion gene, based on western blotting analysis.
[Figure 19A] The figure shows the effects of different inhibitors on viability rate of HCC78 cells.
[Figure 19B] The figure shows the effects of different inhibitors on viability rate of JFCR-168 cells.
[Figure 19C] The figure shows the effects of crizotinib and gilteritinib in xenograft models having JFCR-168 cells transplanted therein.
[Figure 20A] The figure shows that a low dose of gilteritinib exerts an effect in xenograft models using JFCR-028-3 cells.
[Figure 20B] The figure shows that a low dose of gilteritinib exerts an effect in xenograft models using H2228 cells.
[Figure 20C] The figure shows that a low dose of gilteritinib exerts an effect in xenograft models using MCC-003 cells.

Gilteritinib exerts an effect on a tumor having a mutation in an ALK kinase domain and having an acquired ALK-TKI resistance mutation, as disclosed in detail below. Even if resistance to an ALK-TKI is acquired through a bypass pathway, if it is acquired by AXL activation, an effect can be obtained by gilteritinib alone; whereas, if resistance to ALK-TKI is acquired through KRAS, BRAF or EGFR pathway, an effect can be obtained by a combination (of gilteritinib) with a drug suppressing each of the pathways.

If a subject acquires resistance to an existing ALK-TKI by a therapy, whether gilteritinib exerts an effect on a mutation or not, can be determined by detecting a mutation of the ALK gene by an examination method known in the art, such as detection of a mutation by PCR, sequencing and use of an antibody for specifically detecting a mutation. If a mutation is not detected in the ALK gene and resistance is suspected to be developed via a bypass pathway, mutations or activations of KRAS, BRAF and EGFR may be detected by use of a method known in the art such as PCR, sequencing and antibodies.

### 1. Search for drug overcoming ALK-TKI resistant mutation

The inventor has found and reported that some of the EML4-ALK compound mutations having acquired lorlatinib resistance, again become sensitive to existing ALK-TKIs (Non Patent Literature 1). However, the ALK-TKIs already approved do not have an effect on EML4-ALK I1171N+F1174I and I1171N+L1198H compound mutation. Then, to search a drug overcoming these mutations, EML4-ALK I1171N+F1174I or EML4-ALK I1171N+L1198H was introduced into Ba/F3 cells (obtained from Riken BRC) and expressed therein. Using the expression cells and an inhibitor library mostly constituted of available kinase inhibitors, the cell-proliferation inhibitory effect was examined. Note that, the mutant gene-introduced cells to be used later were prepared by integrating a gene having a mutation into a lentiviral vector and introducing the vector into cells. The EML4-ALK fusion genes and mutants thereof to be used later were prepared based on the fusion genes disclosed in Non Patent Literature 1 unless otherwise specified.

Table 1 shows the viability rates of cells when different types of drugs were added to the cells in a concentration of 50 nM. The viable cells were counted 72 hours later in accordance with the CellTiter-Glo assay (Promega). The viability rates of cells are shown based on the number of viable cells when DMSO was added, as 100. As a result, it was confirmed that gilteritinib, which is a therapeutic drug for FLT3 gene mutation-positive acute myeloid leukemia, exerts an antiproliferative effect on EML4-ALK wild type, and I1171N+F1174I and I1171N+L1198H compound mutations. Note that, the drugs used herein were obtained from the following sources:
Crizotinib, brigatinib and lorlatinib: Shanghai Biochempartner,
Gilteritinib: Shanghai Biochempartner or Biovision,
Alectinib and ceritinib: ActiveBiochem,
Entrectinib: Medchem Express,
Afatinib: ChemieTek, and
Trametinib: AdooQ Bioscience.
Brigatinib was dissolved in ethanol and other drugs in DMSO and put in use.

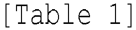

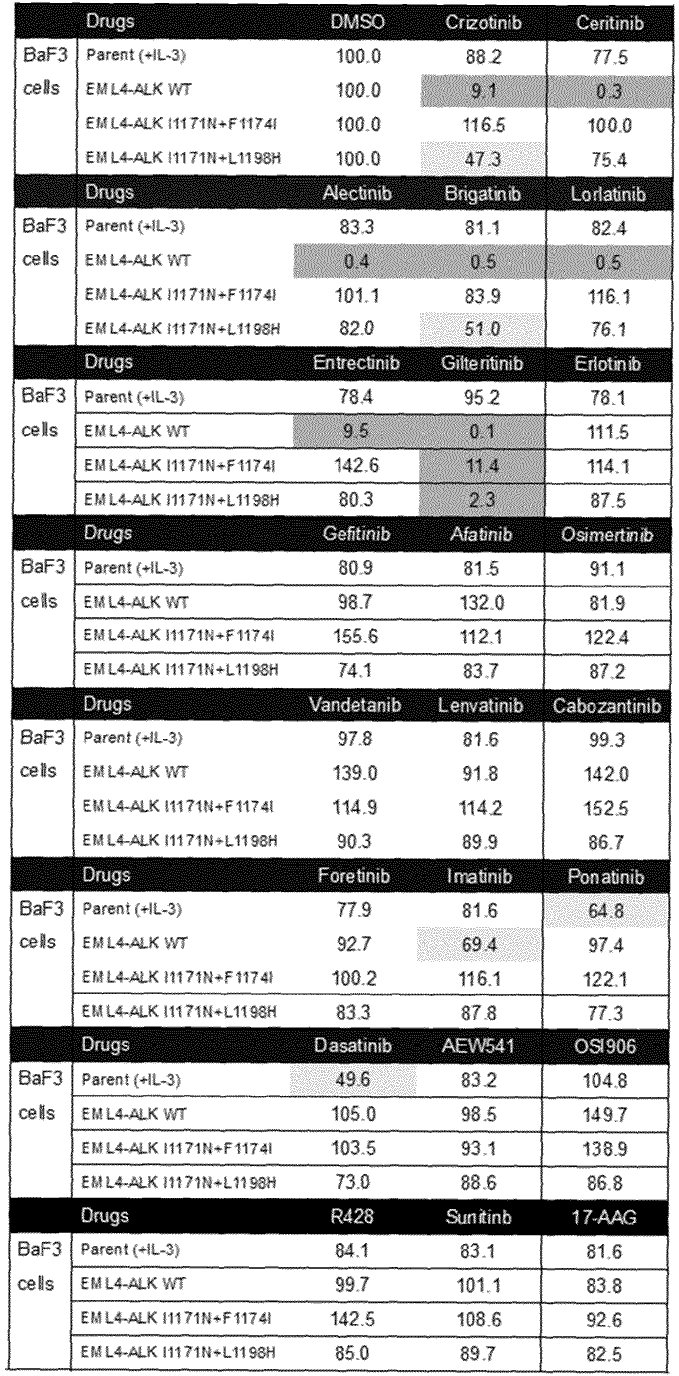

Crizotinib, ceritinib, alectinib, brigatinib and lorlatinib, which have been approved as ALK inhibitors, and additionally entrectinib are confirmed to have an antiproliferative effect on a wild-type ALK fusion gene. However, these drugs did not show strong antiproliferative effect on compound mutations. In contrast, gilteritinib was confirmed to have a strong cell proliferation inhibitory effect on I1171N+F1174I and I1171N+L1198H compound mutations.

### 2. Analysis of action mechanism of gilteritinib

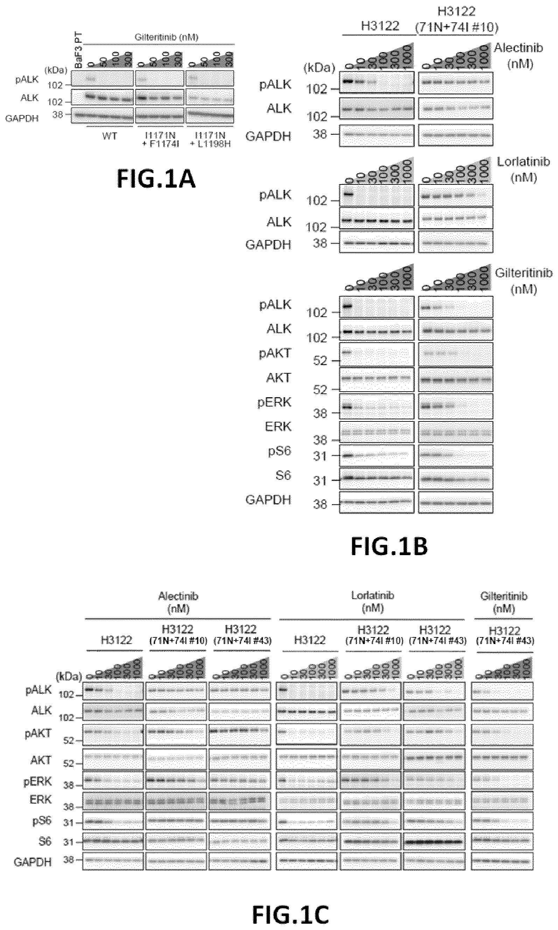

Gilteritinib is a multikinase inhibitor. Then, whether the cell proliferation inhibitory effect of gilteritinib on a compound mutation is produced from the direct action of gilteritinib on a target ALK compound mutation, was analyzed. Ba/F3 in which EML4-ALK wild type, I1171N+F1174I and I1171N+L1198H compound mutations were expressed, were treated with different concentrations of gilteritinib for 3 hours and subjected to western blotting. In this manner, autophosphorylation of ALK was analyzed (Fig.1A). As a result, it was found that gilteritinib inhibits autophosphorylation of the ALK wild type and the compound mutants. In short, it was suggested that gilteritinib directly acts on ALK as a target.

Subsequently, using EML4-ALK-positive non-small cell lung cancer cell line H3122 (obtained from the MGH Cancer Center), whether ALK autophosphorylation is suppressed by gilteritinib, was analyzed. H3122 cells and H3122 having I1171N+F1174I compound mutation overexpressed therein, were separately treated with alectinib, lorlatinib and gilteritinib. The phosphorylation of ALK and phosphorylation of AKT, ERK and S6, which are positioned in the signaling pathway downstream of ALK, were analyzed by western blotting (Fig.1B, C). In H3122 cells (parental cell line), both alectinib and lorlatinib inhibited phosphorylation of ALK and the phosphorylation of the molecules in the signaling pathway downstream of ALK but gilteritinib was only one having an effect of inhibiting phosphorylation of ALK and the molecules in the signaling pathway downstream thereof, at a low concentration, in cells having I1171N+F1174I compound mutation overexpressed therein. Since the degrees of inhibition of phosphorylation of ALK and phosphorylation of the molecules in the signal transduction system downstream thereof have a correlation with the concentration of gilteritinib, it was suggested that gilteritinib directly inhibits ALK.

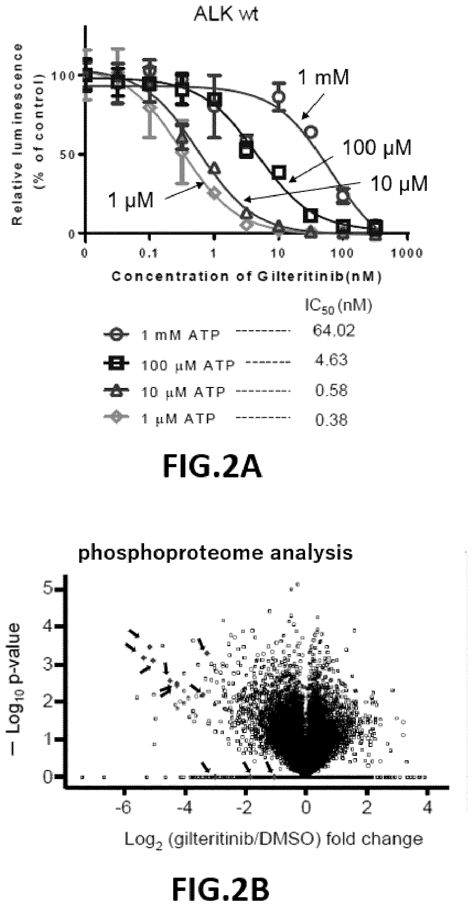

To confirm that gilteritinib directly inhibits ALK, an in-vitro kinase assay was carried out. In the assay, the kinase activity of ALK was measured at different ATP concentrations (Fig.2A). The kinase activity of ALK was measured by the ADP-Glo assay kit (Promega) in the presence of 1 µM to 1 mM ATP. It was found that the kinase activity of ALK is inhibited in an ATP concentration-dependent manner and IC₅₀ value increases in an ATP concentration-dependent manner. More specifically, it was shown that gilteritinib inhibits the kinase activity of ALK competitively with ATP. In other words, it was demonstrated that gilteritinib directly inhibits ALK.

A change in the degree of phosphorylation of a peptide treated with gilteritinib was analyzed by phosphoproteome analysis. ALK-positive lung cancer cells were treated with gilteritinib or DMSO and the cell lysate was treated by a routine method and subjected to phosphoproteome analysis (Fig.2B). The arrows indicate phosphorylated ALK peptides. It was shown that gilteritinib significantly suppresses phosphorylation of ALK. Although data were not shown herein, phosphorylation of ALK adapter proteins, such as IRS1/2, SOS2 and SH2B2, was significantly suppressed by treatment with gilteritinib. These results indicate that gilteritinib directly inhibits ALK competitively with ATP.

### 3. Antiproliferative effect of gilteritinib

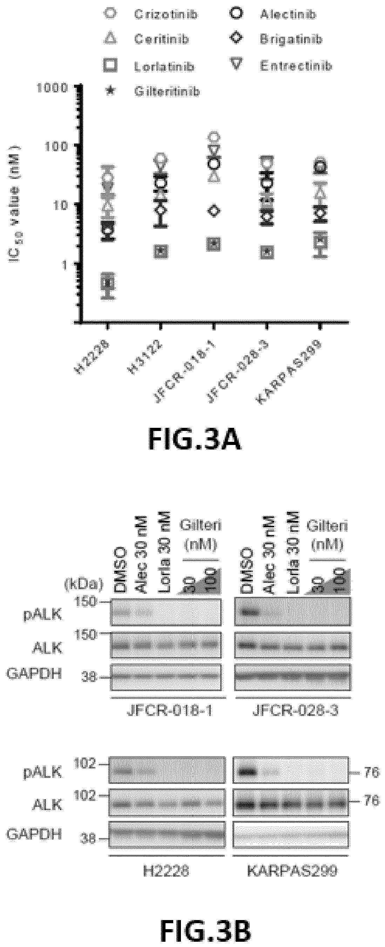

Whether gilteritinib has an antiproliferative effect on non-small cell lung cancer having ALK rearrangement was confirmed. Using EML4-ALK fusion gene-positive non-small cell lung cancer cells H2228 (obtained from ATCC) and H3122 (obtained from the MGH Cancer Center) and primary cultured cells (JFCR-018-1 and JFCR-028-3) derived from patients with ALK fusion gene-positive lung cancer, an assay for viable cells was carried out. Note that, the names of cells having "JFCR" mean the cells established in the Japanese Foundation for Cancer Research. The IC₅₀ values of gilteritinib and ALK-TKIs in individual cell lines are shown in Fig.3A. It was confirmed that gilteritinib has an antiproliferative effect on any cells at a concentration as low as lorlatinib. In addition, gilteritinib exerted an antiproliferative effect on cells of NPM-ALK fusion gene-positive human non-Hodgkin's lymphoma cell line KARPAS299. More specifically, it is shown that gilteritinib has a therapeutic effect on not only non-small cell lung cancer but also ALK gene mutation-positive tumor.

Gilteritinib, alectinib and lorlatinib were separately added to these non-small cell lung cancer cell lines, and then, autophosphorylation was analyzed by western blotting. Gilteritinib inhibited autophosphorylation of ALK in any cell lines (Fig.3B). Although data were not shown herein, gilteritinib suppressed the activation of molecules such as AKT, ERK, S6 and STAT3 of the downstream signaling pathway.

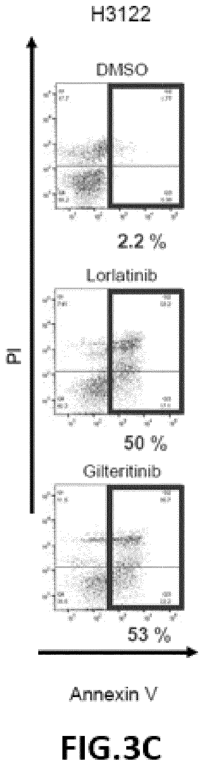

The effect of gilteritinib on apoptosis induction was analyzed by flow cytometry (Fig.3C). To H3122 cells, a solvent DMSO, lorlatinib and gilteritinib were separately added at a concentration of 100 nM. Seventy-two hours later, the cells were stained with annexins V/propidium iodide. The ratio of the cells whose apoptosis was induced was determined. Apoptosis was induced in about 50% of the cells by gilteritinib and lorlatinib.

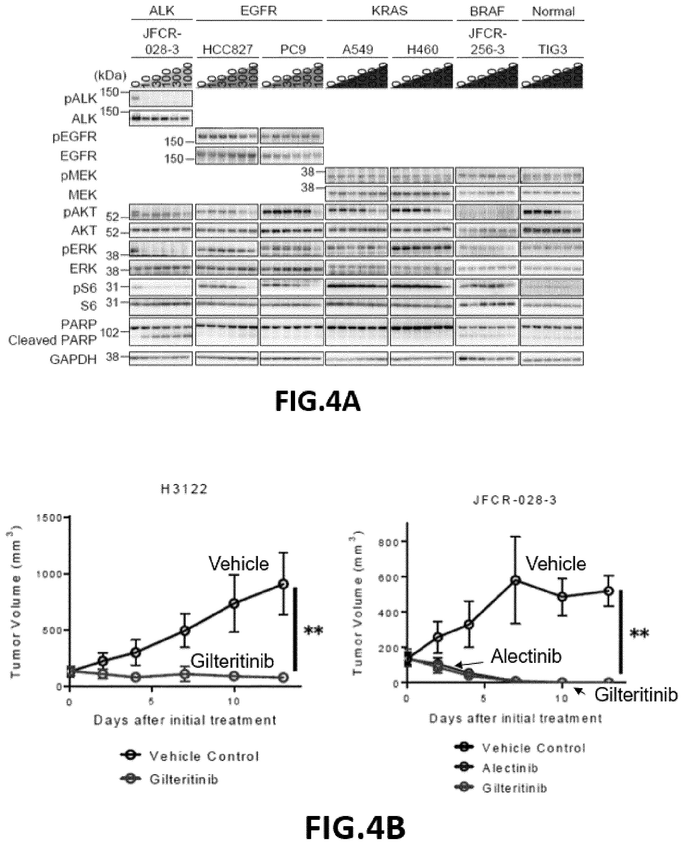

A plurality of non-small cell lung cancer driver genes are present other than ALK. Then, the effects of gilteritinib on non-small cell lung cancer caused by causative genes other than the ALK gene mutation were examined. The HCC827 cells having an EGFR-activated mutation (obtained from ATCC), PC9 (obtained from Riken BRC), KRAS mutation-positive cells A549 (obtained from Riken BRC), H460 (obtained from ATCC), BRAF mutation-positive patient-derived cells JFCR-256-3, and human normal lung fibroblasts TIG-3 (obtained from Riken BRC) were treated with gilteritinib different in concentration. The inhibitory effect on EGFR or the signaling pathway downstream thereof and induction of apoptosis were analyzed by western blotting. Autophosphorylation of ALK and phosphorylation of proteins in the signaling pathway downstream of ALK were suppressed and apoptosis was induced by gilteritinib in the ALK gene mutation-positive cells, JFCR-028-3 cells. However, gilteritinib did not virtually exert an inhibitory effect on EGFR, KRAS, BRAF and any gene mutation-positive cells (Fig.4A).

Based on the confirmation that gilteritinib has an in-vitro antiproliferative effect on ALK gene mutation-positive cancer, whether gilteritinib has an in-vivo antiproliferative effect, was examined. H3122 or JFCR-028-3 cells were transplanted under the skin of BALB/c nu/nu mice. After the volume of a tumor reached about 150 mm3, a solvent alone as control and gilteritinib were administered at a dose of 30 mg/kg. To the mice of the JFCR-028-3 transplanted group, not only gilteritinib but also alectinib was forcibly administered via the oral route at a dose of 30 mg/kg once a day for 5 days per week. The effect was analyzed (each group: n = 6). The volume of a tumor was measured three times per week. It was confirmed that gilteritinib has a significant tumor growth inhibitory effect in the in-vivo test (Fig.4B).

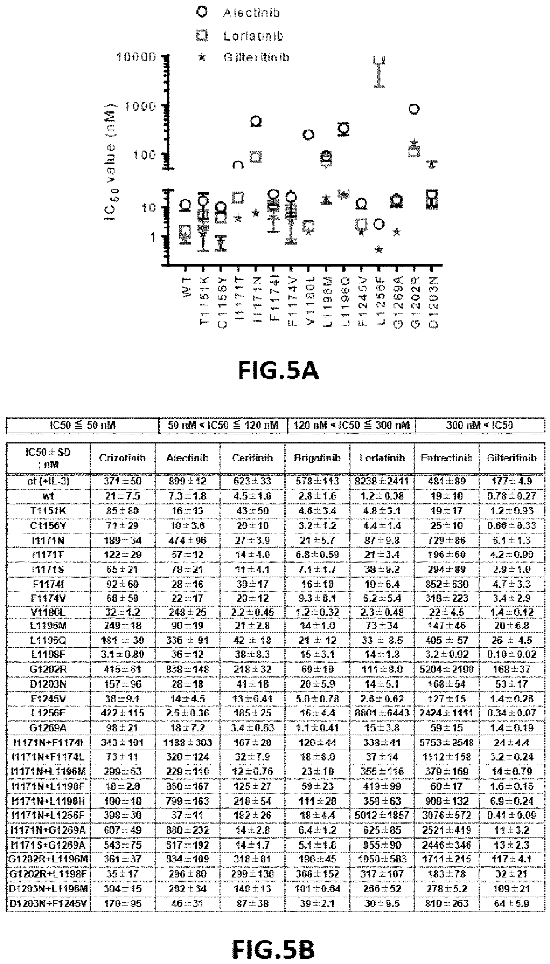

It has been reported that alectinib-resistant patients have gene mutations associated with an amino acid substitution, such as I1171T/N/S, V1180L, G1202R and L1196M, in the ALK kinase domain. An ALK mutant having a single mutation confirmed in a tumor resistant against alectinib was expressed in Ba/F3, and gilteritinib, alectinib and lorlatinib were separately added to the cells. After the cells were cultured for 72 hours, the cell viability rate was calculated in accordance with the CellTiter-Glo assay to obtain IC₅₀ values (Fig.5A).

The IC₅₀ values of gilteritinib were as follows: EML4-ALK I1171T, 4.17 nM; EML4-ALK I1171N, 6.13 nM; EML4-ALK I1171S, 2.86 nM; EML4-ALK V1180L, 1.45 nM; EML4-ALK L1196M, 20.4 nM; and EML4-ALK G1202R, 168 nM. More specifically, the IC₅₀ values of the mutations except G1202R were 30 nM or less. It was demonstrated that gilteritinib has high cell proliferation inhibitory effects on these mutations.

The cell proliferation inhibitory effects on not only these alectinib-resistant mutations but also mutations reported as crizotinib- and ceritinib-resistant mutations were analyzed. As a result, the IC₅₀ values of individual mutants were EML4-ALK C1156Y, 0.66 nM; EML4-ALK F1174V, 3.41 nM; EML4-ALK F1245V, 1.41 nM; EML4-ALK G1269A, 1.39 nM; EML4-ALK T1151K, 1.24 nM; EML4-ALK F1174I, 4.72 nM; EML4-ALK L1196Q, 25.5 nM; and EML4-ALK D1203N, 53.0 nM. It was confirmed that gilteritinib has strong cell proliferation inhibitory effects on the mutations except the D1203N mutation. Gilteritinib exhibited a cell proliferation inhibitory effect (low IC₅₀ value: 0.34 nM) on the lorlatinib-resistant mutation, EML4-ALK L1256F, recently detected by the inventor.

The effects of TKIs (known to be effective for ALK mutants) and gilteritinib on ALK mutants were analyzed. To cell (Ba/F3 cells) models having different mutants expressed therein, crizotinib, alectinib, ceritinib, brigatinib, lorlatinib, entrectinib and gilteritinib, which are known to be effective for ALK-TKIs or ALK mutants, were added, in the same manner as above. Seventy-two hours after the cells were cultured, the cell viability rates were determined in accordance with the CellTiter-Glo assay to obtain IC₅₀ values (Fig.5B). As a result, it was found that gilteritinib has an effect on not only I1171N+F1174I and I1171N+L1198H mutants but also various ALK mutants at a low concentration, compared to ALK-TKIs already approved.

Up to the present, many ALK mutations have been found. Gilteritinib is conceivably effective for combinations of the mutations. For example, with respect to I1171N/T/S mutation, compound mutations with T1151ins, F1174I/L, L1196M, L1198F/H, G1202R, D1203N, F1245V, L1256F and G1269A are presumed. With respect to V1180L mutation, compound mutations with T1151ins, F1174I/L, L1196M, L1198F/H, G1202R, D1203N, F1245V, L1256F and G1269A are presumed. With respect to G1202R mutation, compound mutations with F1174I/L, L1196M, L1198F/H and G1269A are presumed. With respect to L1196M mutation, compound mutations with F1174I/L, L1198F/H, G1202R, D1203N, F1245V, L1256F and G1269A are presumed. Gilteritinib conceivably exerts an effect on these compound mutations. Note that I1171N/T/S herein means a mutant where I at the 1171st position is replaced with N, T or S. The same rule applies to other mutants.

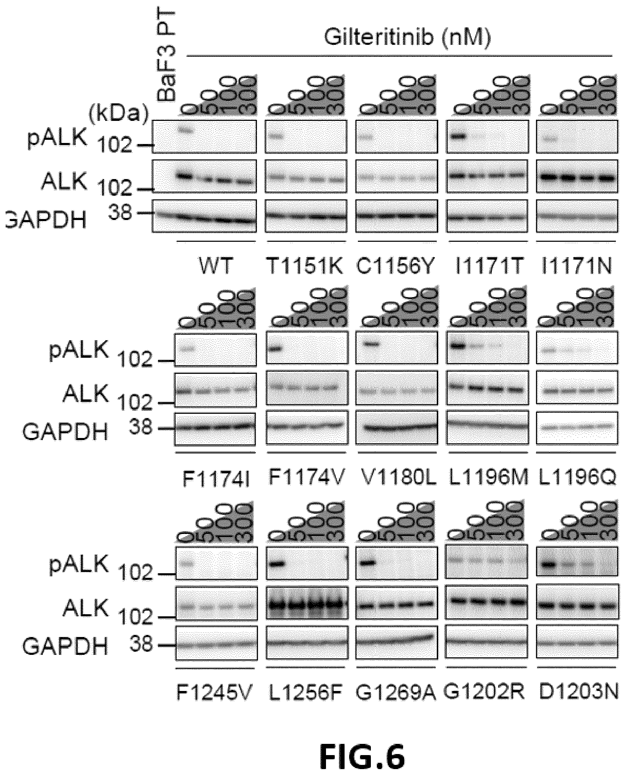

Autophosphorylation of ALK gene mutants having these single mutations by treatment with gilteritinib was analyzed by western blotting (Fig.6). The autophosphorylation of the mutants except G1202R and D1203N was completely suppressed by treatment with 50 nM gilteritinib in conformity with the antiproliferative effect by gilteritinib.

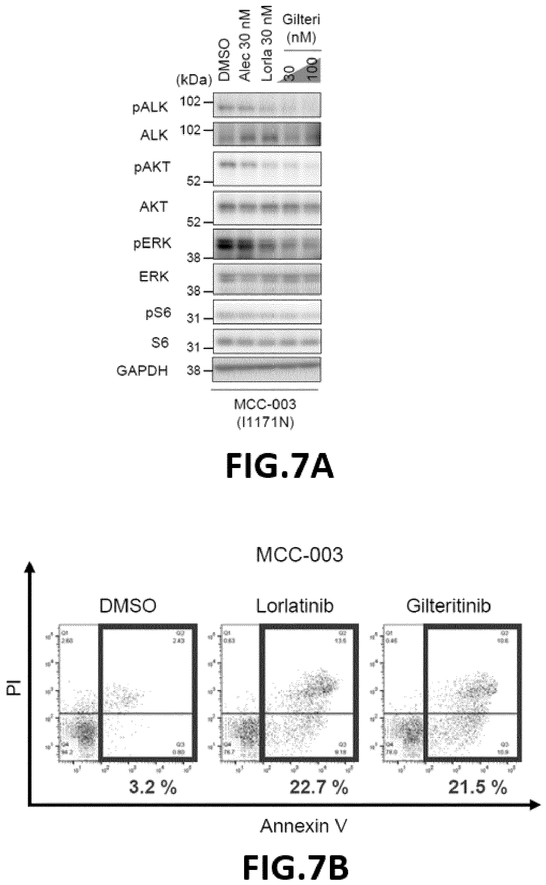

Using not only a cell model using Ba/F3 but also MCC-003 cells (established at the Japanese Foundation for Cancer Research from a patient's specimen from the Miyagi prefecture cancer center) derived from a patient having acquired resistance to alectinib and having EML4-ALK-I1171N mutation, the inhibitory effect of gilteritinib was examined. MCC-003 cells were separately treated with alectinib, lorlatinib and gilteritinib for 6 hours and subjected to western blotting to analyze phosphorylation of ALK and the signaling pathway downstream thereof (Fig.7A). As a result, it was found that gilteritinib suppresses autophosphorylation of ALK and activation of the signaling pathway downstream thereof in MCC-003 cells.

It was further confirmed that gilteritinib induces apoptosis of MCC-003 cells (Fig.7B). MCC-003 cells were separately treated with DMSO (solvent) and 100 nM lorlatinib and 100 nM gilteritinib. Seventy-two hours later, the MCC-003 cells were stained with annexins V/propidium iodide. The ratio of cells whose apoptosis was induced was determined. Apoptosis was induced in about 20% of the cells treated separately with gilteritinib and lorlatinib.

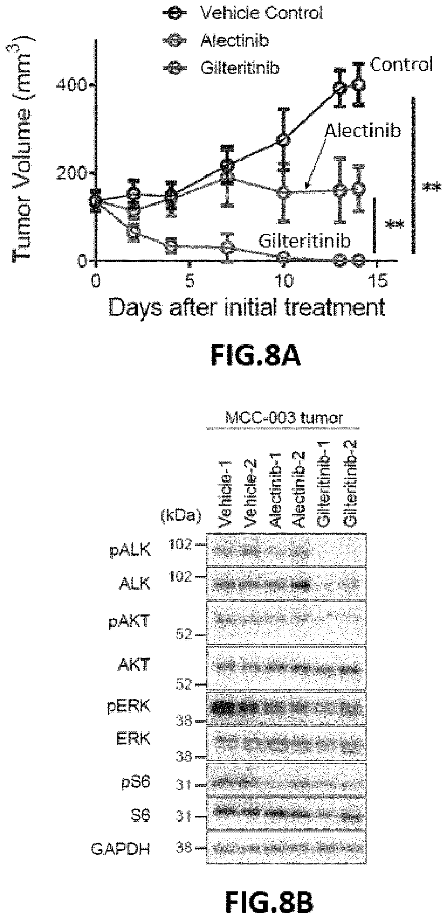

MCC-003 cells were transplanted into BALB/c nu/nu mice. Using the xenograft models, the effect of gilteritinib was analyzed. MCC-003 cells were transplanted under the skin of the mice. After the average tumor volume reached 150 mm3, a solvent (as a control), alectinib or gilteritinib was given to the mice by forced oral administration at a dose of 30 mg/kg once a day for 5 days per week. Eight mice were used in each group in the experiment. The volume of a tumor was measured three times per week (Fig.8A). In a group treated with gilteritinib, significant shrinkage of a tumor was observed. In contrast, in a group treated with alectinib, significant shrinkage of a tumor was not observed. Note that, a decrease in body weight was not observed up to Day 15 in all groups.

In a tumor of the MCC-003 cells transplanted into mice, whether autophosphorylation of ALK and activation of the signaling pathway downstream thereof is suppressed by administration of gilteritinib, was analyzed by western blotting (Fig.8B). Suppression of autophosphorylation of ALK and activation of the signaling pathway downstream thereof were observed by administration of gilteritinib, whereas a significant suppression was not observed by administration of alectinib.

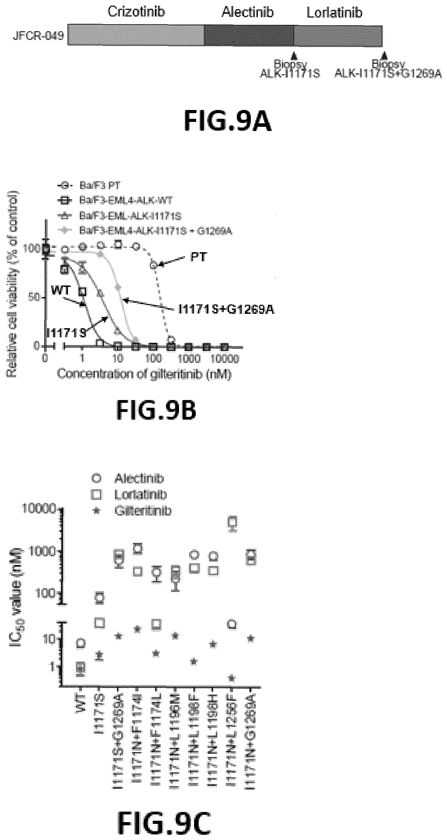

Lorlatinib has a therapeutic effect on most of the first-generation and second-generation ALK-TKI resistant single genetic mutations. However, the inventor has already reported that lorlatinib resistance is acquired due to a compound mutation developed in the ALK kinase domain (Non Patent Literatures 2 and 3). The inventor also found a case where ALK I1171S+G1269A compound mutation developed as a lorlatinib-resistant mutation (Fig.9A). Patient JFCR-049 received chemotherapy (4 cycles of cisplatin/pemetrexed/bevacizumab), followed by a treatment with crizotinib, alectinib and lorlatinib. The resistant mutation was detected in metastasis in the liver after the treatment with lorlatinib. I1171S+G1269A compound mutation was sensitive to second-generation ALK-TKIs, ceritinib and brigatinib. A patient's tumor shrank by treatment with ceritinib.

Since gilteritinib has an effect on a mutation having acquired resistance to the first-generation and second-generation ALK-TKIs, as shown in the above, whether gilteritinib is effective for a lorlatinib-resistant compound mutation was evaluated. EML4-ALK I1171S+G1269A compound mutation was introduced into Ba/F3 cells and expressed therein. The effect of gilteritinib on the viability rate of the cells was analyzed (Fig.9B). EML4-ALK wild type, an EML4-ALK I1171S single gene mutant and an EML4-ALK I1171S+G1269A compound mutant were expressed in Ba/F3 cells. Gilteritinib was added to culture mediums. Seventy-two hours later, the viability rates of the cells were evaluated by the CellTiter-Glo assay. The IC₅₀ value of the single gene mutation (I1171S) was 2.86 nM; the IC₅₀ value of the compound mutation (I1171S+G1269A) was 23.5 nM. It was confirmed that gilteritinib has a cell proliferation inhibitory effect on both the single gene mutation and the compound gene mutation.

Furthermore, I1171N compound mutations were expressed in Ba/F3 cells and the effects of alectinib, lorlatinib and gilteritinib on cell proliferation were analyzed. The IC₅₀ values of gilteritinib in compound mutations with I1171N were all 30 nM or less (I1171N+F1174I, 23.5 nM; I1171N+F1174L, 3.15 nM; I1171N+L1196M, 14.0 nM; I1171N+L1198F, 1.64 nM; I1171N+L1198H, 6.95 nM; I1171N+L1256F, 0.41 nM; and I1171N+G1269A, 11.4 nM); that is, gilteritinib exerted a high cell proliferation inhibitory effect (Fig.9C, Fig.5B) .

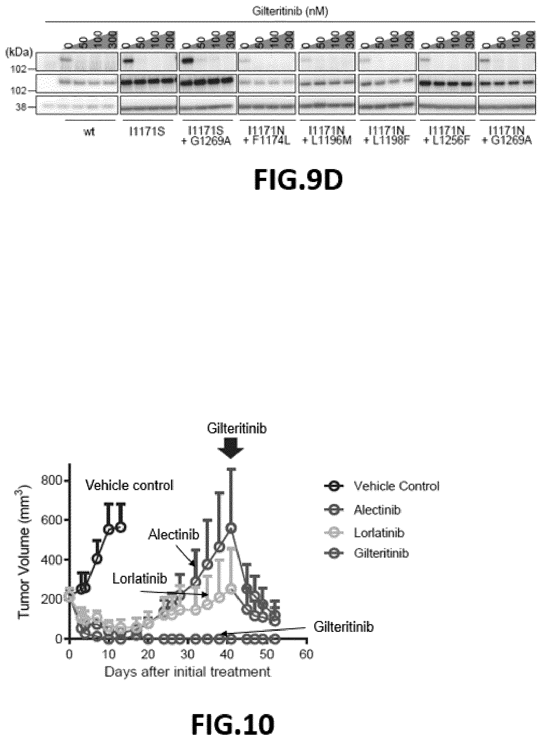

The effects of gilteritinib on autophosphorylation of the first-generation and second-generation ALK-TKI-resistant mutations were analyzed. A I1171S single gene mutation and compound mutations were expressed in Ba/F3 cells. The cells were treated with gilteritinib. Three hours later, the cells were collected and subjected to western blotting analysis (Fig.9D). The autophosphorylation of ALK in any one of the mutants analyzed was inhibited by gilteritinib.

Next, JFCR-028-3 cells having a lorlatinib-resistant compound mutation, ALK I1171N+F1174I, expressed therein, were transplanted into mice. The effect of gilteritinib was evaluated in the in-vivo model. JFCR-028-3 having EML4-ALK I1171N+F1174I expressed therein were transplanted into BALB/c nu/nu mice. After the volume of a tumor reached 200 mm3, a solvent alone, alectinib (30 mg/kg), lorlatinib (5 mg/kg) or gilteritinib (30 mg/kg) was given to the mice by forced oral administration once a day for 5 days per week (each group n = 6). On Day 41, to alectinib- and lorlatinib-treated groups, administration of gilteritinib (30 mg/kg, once a day, 5 days per week, forced oral administration) was initiated in place of administration of alectinib and lorlatinib, and the experiment was continued. The volume of a tumor was measured three times per week (Figure 10).

In the groups treated with alectinib and lorlatinib, regrowth of a tumor was observed in a short period of time; whereas complete shrinkage of a tumor was observed for 50 days or more in the group treated with gilteritinib. In the groups treated with alectinib and lorlatinib, gilteritinib was administered after the regrowth of a tumor was observed. As a result, rapid shrinkage of the tumor was observed. This result demonstrates that gilteritinib has a high possibility of exerting an effect on a tumor having acquired resistances against ALK-TKIs presently approved.

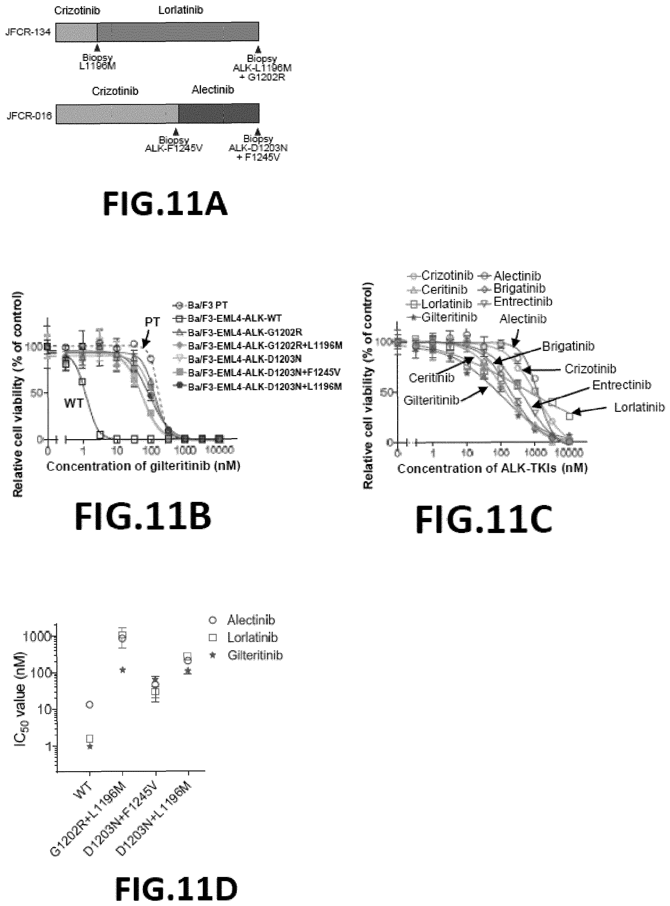

Examples of a mutation having acquired resistances against ALK-TKIs including lorlatinib include ALK L1196M+G1202R and D1203N+F1245V compound mutations. In a patient, JFCR-134, after treatments with crizotinib and lorlatinib, 1196M+G1202R was detected. In a patient, JFCR-016, after treatments with crizotinib and alectinib, a D1203N+F1245V compound mutation was detected (Fig.11A). It has been reported that a L1196M+D1203N compound mutation has acquired resistance to all ALK-TKIs clinically applied (Non Patent Literature 4). Based on the report, whether gilteritinib has a cell proliferation inhibitory effect on these three compound mutations, was examined.

EML4-ALK wild type, the three compound mutations having acquired resistance to existing ALK-TKIs, and G1202R and D1203N single mutations were expressed in Ba/F3 cells. The effects of gilteritinib on the viability rates of the cells were examined (Fig.11B). As a result, except an ALK wild type relatively high concentrations of gilteritinib were required for inhibiting the three compound mutations or G1202R and D1203N single mutations.

MR347 cells established from a patient with ALK-TKI-resistant non-small cell lung cancer have an EML4-ALK-D1203N+L1196M compound mutation. The effects of ALK-TKIs and gilteritinib on the viability rate of the MR347 cells were examined (Fig.11C). None of the drugs exerted a significant inhibitory effect on the viability rate of the cells.

Compound mutations D1202R+L1196M, D1203N+F1245V and D1203N+L1196M were expressed in Ba/F3 cells and the IC₅₀ values of alectinib, lorlatinib and gilteritinib were determined (Fig.11D). The IC₅₀ of gilteritinib for cell viability rate were D1202R+L1196M, 117 nM; D1203N+F1245V, 64 nM; and D1203N+L1196M, 109 nM. Different from the compound mutations including I1171N/S previously mentioned, gilteritinib did not exert a significant cell proliferation inhibitory effect on these genetic mutations at a low concentration.

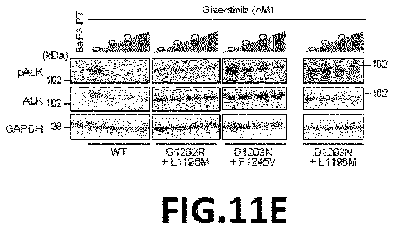

Whether gilteritinib suppresses autophosphorylation of ALK with respect to these compound mutations, was examined (Fig. 11E) . As a result, gilteritinib did not virtually suppress the autophosphorylation of ALK. As mentioned above, gilteritinib is effective for a great many resistant mutations compared to existing ALK-TKIs, but a sufficient effect thereof on some of the above mutations cannot be expected.

### 4. Effect of gilteritinib on bypass mechanism involved in ALK-TKI resistance

The drug resistances of ALK fusion gene-positive cancer are roughly divided into an ALK-independent drug resistance, that is, activation of an ALK bypass pathway such as EGFR, cMET, KRAS, BRAF and AXL, and an ALK-dependent mechanism due to a second mutation produced in ALK. As examined up to here, it was found that gilteritinib has an effect on a lot of mutations in the ALK-dependent pathway. However, the acquisition of resistance by a bypass pathway has become a concern. Then, whether the bypass pathway for acquiring resistance without involving ALK can be suppressed by gilteritinib, was examined. Note that, although data were not shown, with respect to activation of AXL by Gas6 expression induction, gilteritinib exerts an effect on resistant cells without being affected.

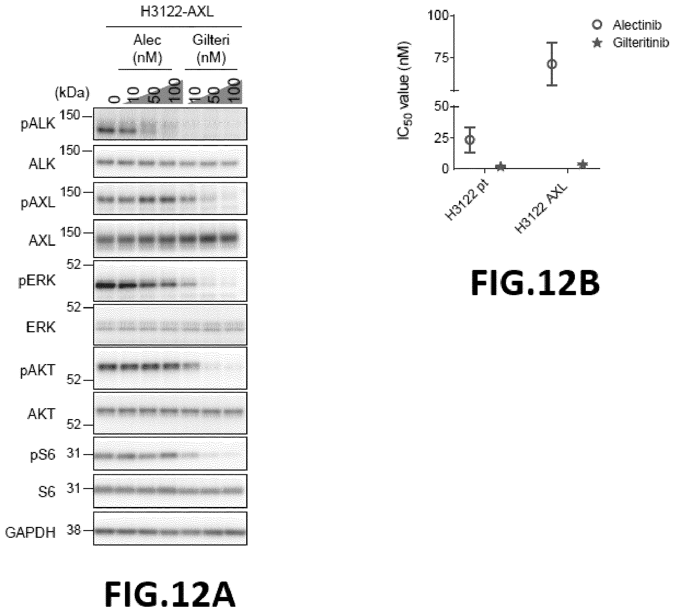

In EGFR-positive lung cancer, it has been reported that a decrease of response to EGFR-TKI correlates with activation of AXL (Non Patent Literature 5). Then, whether the activations of AXL and the signaling pathway downstream of AXL are suppressed by gilteritinib, was analyzed by western blotting. AXL was overexpressed in EML4-ALK-positive non-small cell lung cancer cell line H3122, and the cells were separately treated with alectinib and gilteritinib and subjected to western blotting to analyze AXL and the signaling pathway downstream of AXL (Fig.12A). Gilteritinib suppressed the activations of AXL and signaling pathway downstream thereof, MAPK signal pathway and PI3-AKT signaling pathway. However, AXL and the signaling pathway downstream thereof were not inhibited by the addition of alectinib.

The IC₅₀ values of alectinib and gilteritinib for viability rate were obtained. As a result, the IC₅₀ value of alectinib was significantly high when AXL was overexpressed but the IC₅₀ value of gilteritinib was 5 nM or less even if AXL was overexpressed. It was found that gilteritinib is virtually not affected by overexpression of AXL.

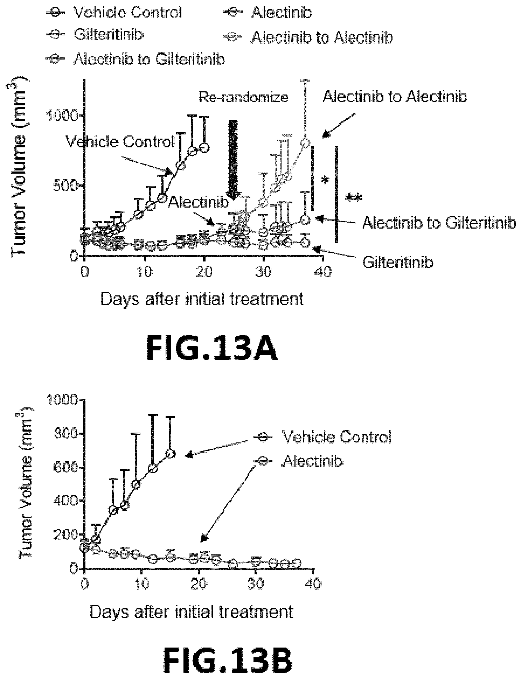

H3122 cells having AXL overexpressed therein were transplanted under the skin of BALB/c nu/nu mice. After the average tumor volume reached 150 mm3, the mice were divided into groups. As a control group a solvent alone, and as treatment groups alectinib (30 mg/kg) and gilteritinib (30 mg/kg) were separately given to the mice by forced oral administration once a day for 5 days per week. On Day 25, the mice of the alectinib treatment group were divided at random into two groups: a group of the mice to which alectinib is continuously administered and a group of the mice to which gilteritinib is administered in place of alectinib (each group; n = 6). The tumor growth inhibitory effect was observed in the beginning in the alectinib administration group but tumor growth was observed in about three weeks. In contrast, in the gilteritinib administration group or an administration group of alectinib followed by gilteritinib, the growth of a tumor was suppressed (Fig.13A). The fact that gilteritinib exerted a tumor growth inhibitory effect in the administration group of alectinib followed by gilteritinib demonstrates that gilteritinib has a growth inhibitory effect on a tumor having acquired resistance to alectinib. The effect of alectinib on a parental cell line, H3122, was examined in xenograft models in the same manner as above. As a result, the proliferation of H3122 cells was completely suppressed by alectinib (Fig.13B).

Next, the KRAS signaling pathway known as a bypass pathway was examined. It has been reported that induction of lineage switching and activation of the MAPK signaling pathway are important as the ALK-TKI-resistant mechanism (Non Patent Literatures 6 and 7). It has been also reported that mutations such as KRAS G12C and KRAS G13D were present in the recurrence cases of an EML4-ALK-positive tumor after treatment with a plurality of ALK-TKIs (Non Patent Literature 8). Then, whether gilteritinib exerts an effect particularly on KRAS G12C mutation, was examined.

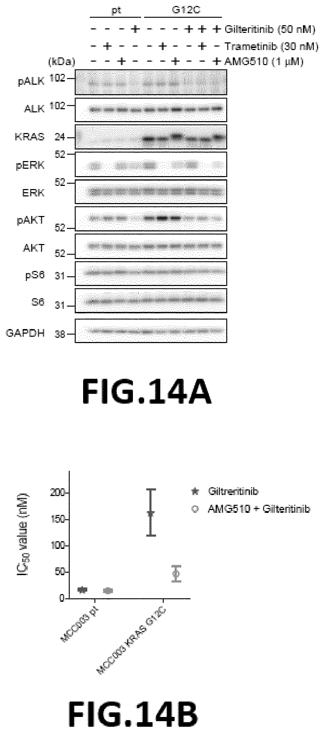

Whether the activity of the signaling pathway downstream of KRAS is inhibited, was analyzed by western blotting using MCC-003 cells having KRAS G12C expressed therein. Whether activation of the KRAS signaling pathway is inhibited by the addition of gilteritinib, trametinib which suppresses the MAPK signaling pathway by inhibiting MEK, and AMG510 (obtained from Medchem Express) which is a KRAS G12C specific inhibitor, alone or in combination, was analyzed (Fig.14A). As a result, it was found that gilteritinib was not able to completely suppress the KRAS signaling pathway. The IC₅₀ value of gilteritinib for viability rate was determined and showed high value (Fig.14B).

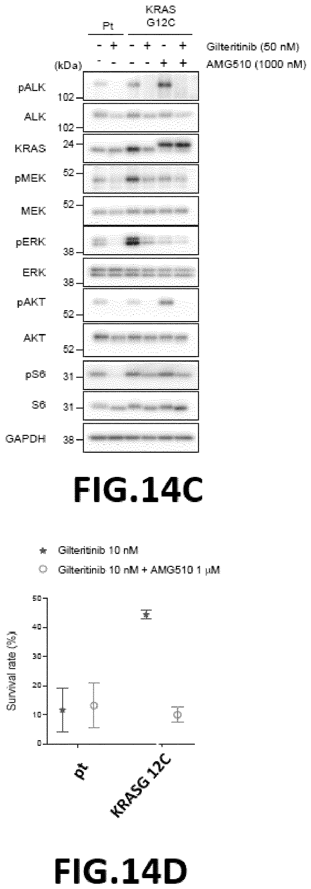

The same analysis as above was carried out by western blotting using JFCR-028-3 cells having KRAS G12C expressed therein. In the case where KRAS G12C was introduced into JFCR-028-3 cells, it was confirmed that activation of the signaling pathway downstream of KRAS was slightly suppressed even by gilteritinib alone (Fig.14C). In the case where gilteritinib was added alone, the IC₅₀ for viability rate was high (Fig.14D).

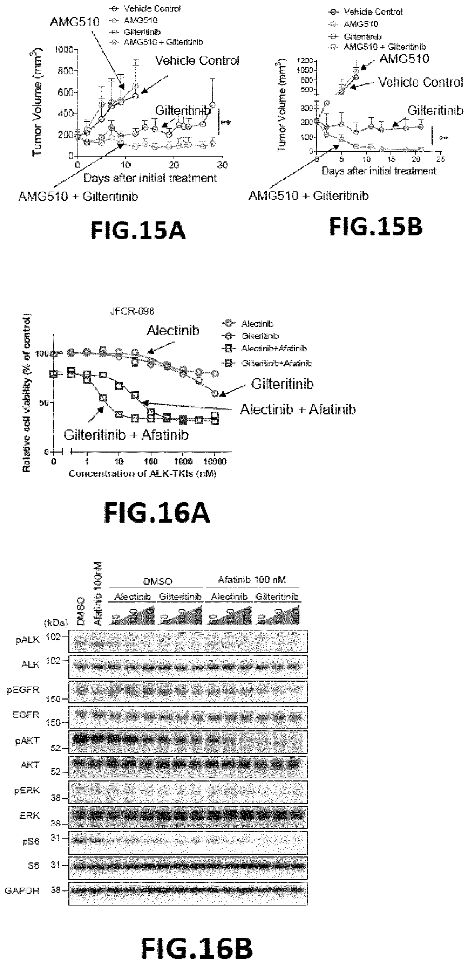

In light of these results, to overcome resistance via KRAS G12C, it was considered necessary that a KRAS G12C specific inhibitor and gilteritinib are used in combination. Then, the use of AMG510 and gilteritinib in combination was tested in in-vivo models. MCC-003 cells having KRAS G12C expressed therein were transplanted under the skin of BALB/c nu/nu mice. When the average tumor volume reached 175 mm3, the mice were divided into groups. As a control group a solvent alone and as test groups AMG510 (100 mg/kg), gilteritinib (30 mg/kg), and AMG510 and gilteritinib in combination were separately given to the mice by forced oral administration once a day for 5 days per week (each group: n = 6). The volume of a tumor was measured 5 times per week (Fig.15A). JFCR-028-3 cells having KRAS G12C expressed therein were subjected to the same experiment as above (Fig.15B). In both cases (MCC-003 cells and JFCR-028-3 cells), no complete tumor growth inhibitory effect was confirmed when MG510 alone and gilteritinib alone were administered. The tumor growth inhibitory effect was observed only when AMG510 and gilteritinib were administered in combination. The effect of gilteritinib on KRAS mutation was examined herein but it is conceivable that the effect is exerted on the bypass pathway via BRAF. BRAF is a molecule positioned downstream of KRAS and activating mutation of BRAF was not only observed as a driver oncogene in melanoma, lung cancer and colon cancer, but also found occasionally in acquired resistant cases of ALK- and EGFR-positive lung cancers. In the cases of ALK inhibitor resistance due to the introduction of BRAF mutation, the use of gilteritinib and a BRAF inhibitor or a drug inhibiting MEK, which is a signaling molecule positioned downstream thereof, in combination, leads to overcoming the resistance.

Next, the EGFR signaling pathway known as a bypass pathway was analyzed. JFCR-098 cells are cells established from a patient who acquired ALK-TKI resistance through the EGFR pathway. JFCR-098 cells were treated with alectinib, gilteritinib, and a combination of each of them and afatinib (EGFR inhibitor), and the effects of these on cell viability rate were analyzed (Fig.16A). It was confirmed that a combination use of alectinib or gilteritinib and the EGFR inhibitor produces a significant cell proliferation inhibitory effect. In particular, the combination use of gilteritinib and afatinib strongly suppressed cell proliferation. The activation of the downstream signaling pathway, which was analyzed by western blotting, was effectively suppressed by the combination use of afatinib and gilteritinib (Fig.16B) .

### 5. Effect of gilteritinib on ROS1 and NTRK fusion gene

In non-small cell lung cancer, various driver gene mutations were identified. One of them is the rearrangement of ROS1 or NTRK gene. As NTRKs, three types of genes: NTRK1/2/3, are known. Each of them is involved in the clinical state of cancer as a fusion gene. The term of "NTRK fusion gene" simply used herein means any one of NTRK1/2/3 fusion genes. The rearrangement of ROS1 gene occupies about 1% of non-small cell lung cancer and the rearrangement of NTRK gene occupies about 0.1% of non-small cell lung cancer. The tyrosine kinase domains of ROS1 and NTRK are structurally analogous to the kinase domain of ALK. From this, it is known that a plurality of ALK-TKIs have an inhibitory effect on the kinase activities of ROS1 and NTRK. Then, the effect of gilteritinib was examined by cell models using Ba/F3 cells.

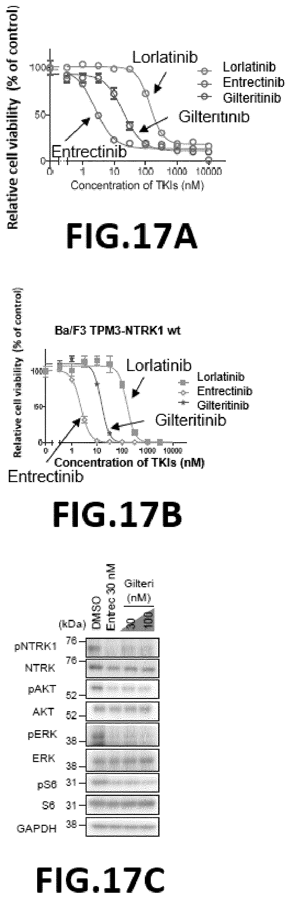

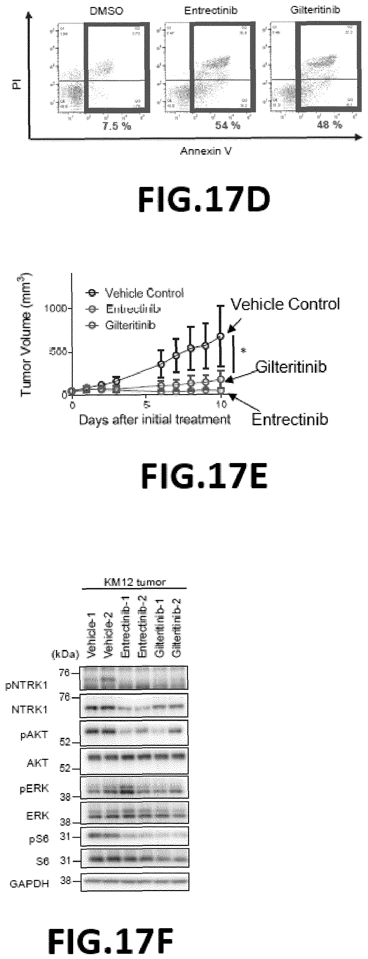

The effect of gilteritinib on viability rate was analyzed in TPM3-NTRK1 fusion gene-positive colon cancer cell line KM12 and Ba/F3 cells having a TPM3-NTRK1 fusion gene introduced therein (Fig.17A, Fig.1 7B). The IC₅₀ value of KM12 by gilteritinib treatment was 30 nM or less and the IC₅₀ value of Ba/F3 having a TPM3-NTRK1 fusion gene expressed therein was 13.3 nM. Both of the cells were highly sensitive to gilteritinib. Note that, entrectinib is a kinase inhibitor used as an inhibitor for NTRK and ROS1 fusion genes.

The downstream signaling pathway was analyzed by western blotting using KM12 cells (Fig.17C). In conformity with the viability rate, gilteritinib suppressed activations of NTRK1 and the signaling pathway downstream thereof. Furthermore, the effect of gilteritinib on apoptosis was analyzed (Fig.17D). Gilteritinib induced apoptosis in about 50% of the cells, similarly to entrectinib.

The effect of gilteritinib was analyzed in xenograft models using KM12 cells (Fig.17E). The KM12 cells were transplanted under the skin of BALB/c nu/nu mice. When the average volume of tumors reached 100 mm3, the mice were divided into groups. As a control group, a solvent alone and as test groups, entrectinib (30 mg/kg) and gilteritinib (30 mg/kg) were separately given to the mice by forced oral administration once a day for 10 days (each group n = 6). The volume of a tumor was measured three times per week. It was found that gilteritinib suppresses the growth of tumors, similarly to entrectinib. Furthermore, activations of NTRK1 and the signaling pathway downstream thereof in tumors were suppressed by gilteritinib (Fig,17F).

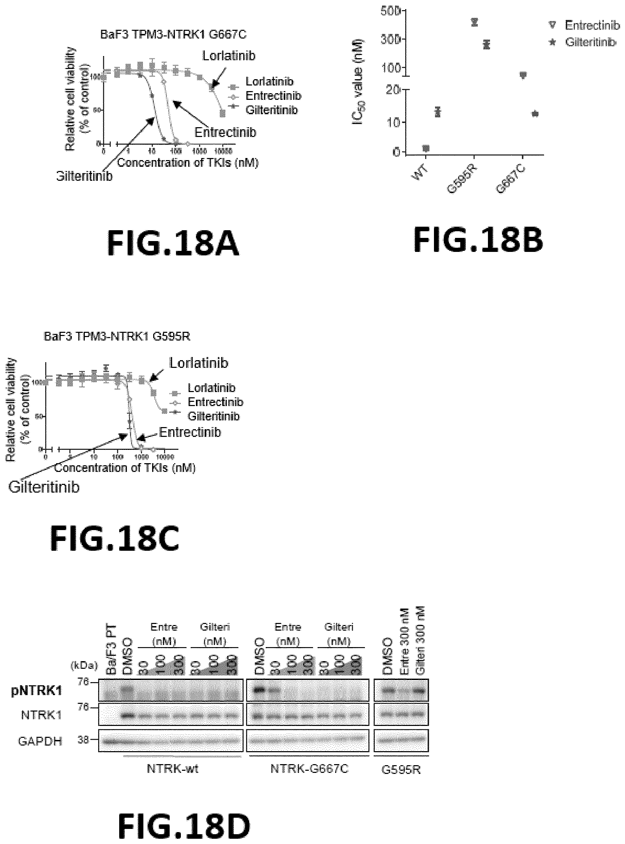

An NTRK gene fusion having a mutation was examined. NTRK1 G667C and G595R are mutations first reported as entrectinib-resistant mutations. It has been reported that drugs such as ponatinib are effective for a NTRK1 G667C mutation but none of the drugs has been approved. Whether gilteritinib has an effect on the mutation, was analyzed. Ba/F3 cells having TPM3-NTRK1-G667C expressed therein were treated separately with gilteritinib, entrectinib and lorlatinib and cell viability rates were analyzed (Fig.18A). The G667C mutation was more sensitive to gilteritinib than the wild type and the IC₅₀ value thereof was 12.7 nM (Fig.18B, Fig.18C). However, neither gilteritinib nor entrectinib was effective for a G595R mutant for viability rate (Fig.18B). The effect on NTRK1 phosphorylation was analyzed. As a result, it was confirmed that gilteritinib has an effect of suppressing phosphorylation of NTRK1 in the G667C mutant (Fig.18D).

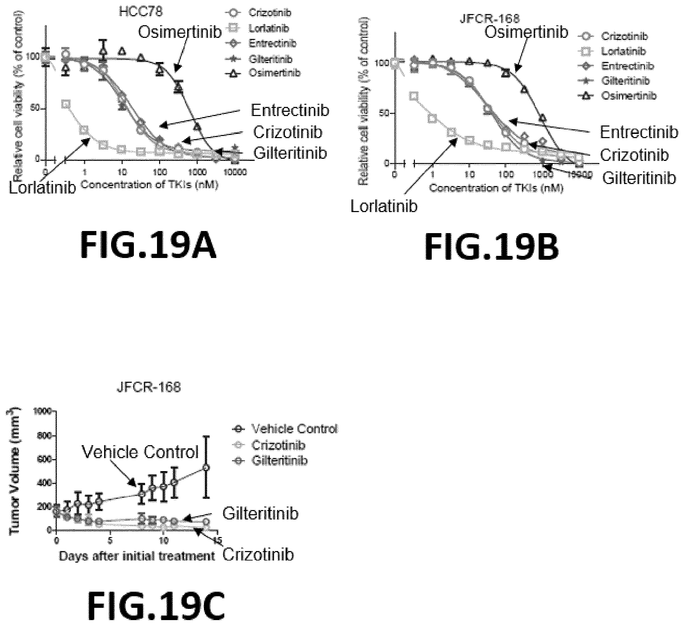

Next, the effect on the ROS1 fusion gene was analyzed. HCC78 cells are cancer cells having the SLC34A2-ROS1 fusion gene; whereas JFCR-168 cells are cancer cells having the CD74-ROS1 fusion gene. Even if either one of the HCC78 cells and JFCR-168 cells were used, the cell proliferation inhibitory effect of gilteritinib was confirmed (Fig.19A, Fig.19B). In xenograft models using JFCR-168 cells, the effect of gilteritinib was analyzed (Fig.19C). JFCR-168 cells were transplanted under the skin of SCID-Beige mice. When the average volume of tumors reached 150 mm3, the mice were divided into groups. As a control group a solvent alone, and as test groups crizotinib (100 mg/kg) and gilteritinib (30 mg/kg) were separately given to the mice by forced oral administration once a day for 5 days per week (each group n = 3). The volume of a tumor was measured three times per week. It was confirmed that both gilteritinib and crizotinib have a significant cytoreductive effect.

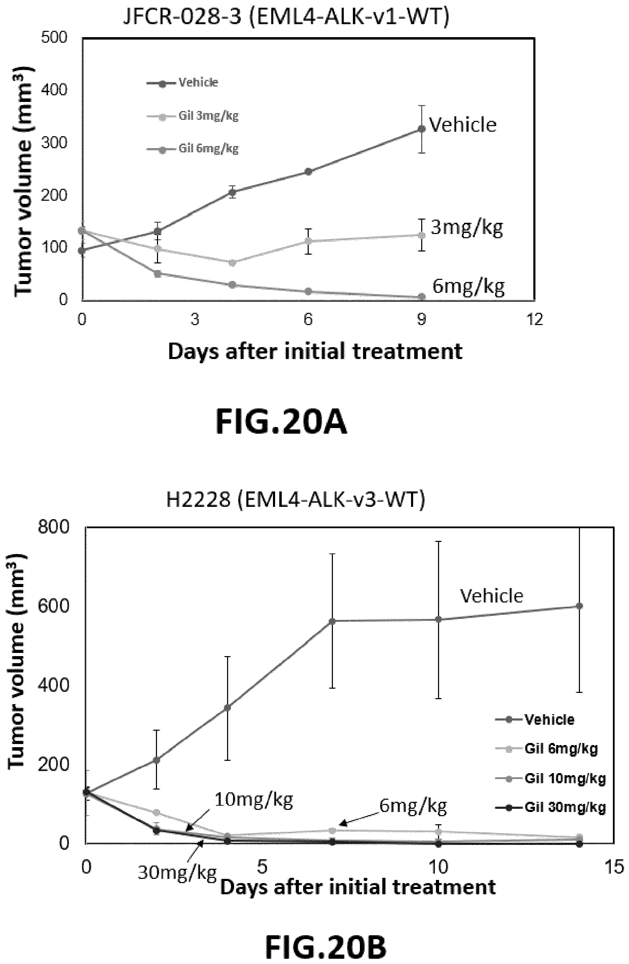

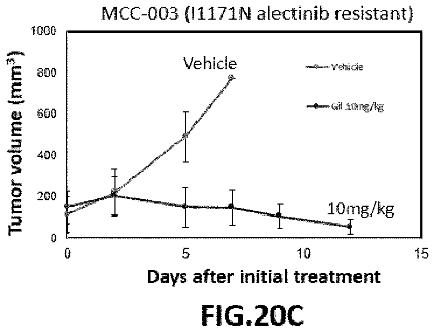

In the experiment using the xenograft models, gilteritinib (30 mg/kg) was administered and the effect thereof was analyzed. Then, whether gilteritinib exerts an effect even if it was given in a low dose, was examined. JFCR-028-3 cells, H2228 cells and MCC-003 cells were separately transplanted under the skin of BALB/C nu/nu mice. When the average diameter of tumors reached 150 mm3, a solvent (control) alone or gilteritinib was given by forced oral administration once a day, and the effect thereof was analyzed (Fig.20). The kinase domain of the ALK gene in JFCR-028-3 cells and H2228 cells are wild type; whereas that of alectinib-resistant MCC-003 cells has an I1171N mutation.

In xenograft models using JFCR-028-3 cells, even if gilteritinib (3 mg/kg) was administered, proliferation of tumor cells was not observed and had a significant difference compared to a control where DMSO was administered. In the 6 mg/kg administration group, shrinkage of a tumor was observed and complete shrinkage of the tumor was confirmed on Day 9 after administration of gilteritinib (Fig.20A). In xenograft models using H2228 cells, gilteritinib was administered at a dose of 6 mg/kg, 10 mg/kg and 30 mg/kg. As a result, shrinkage of a tumor was confirmed in any one of the doses (Fig.20B). In xenograft models using alectinib-resistant MCC-003 cells, gilteritinib (10 mg/kg) was administered. As a result, shrinkage of a tumor was confirmed (Fig.20C).

As mentioned above, it was found that gilteritinib has an effect on tumors having acquired resistances against first-generation and second-generation ALK-TKIs and particularly has an effect on the compound mutation for which an effective drug has not been present. Furthermore, ALK-TKI-resistant cancer having a fusion gene such as NTRK and ROS1, and ALK-TKI-resistant cancer via AXL can be effectively overcome by gilteritinib alone, and ALK-TKI resistance via a bypass pathway such as KRAS, BRAF and EGFR, can be effectively overcome by combination therapy.

## Claims

1. A pharmaceutical composition for treating an ALK fusion gene-positive tumor, containing gilteritinib as an active ingredient.

2. The pharmaceutical composition for treating an ALK fusion gene-positive tumor according to claim 1, wherein the ALK fusion gene-positive tumor is a tumor having a wild type ALK or acquired resistance to a first-generation, a second-generation of ALK-TKIs or lorlatinib.

3. The pharmaceutical composition for treating an ALK fusion gene-positive tumor according to claim 1 or 2, wherein the ALK fusion gene-positive tumor is compound mutations with I1171N, I1171S, V1180L, L1196M or C1156Y.

4. The pharmaceutical composition for treating an ALK fusion gene-positive tumor according to any one of claims 1 to 3, wherein the ALK fusion gene-positive tumor is an ALK fusion gene having at least two compound mutations including mutations: I1171N/S/T, F1174I/L, L1196M, L1198F/H, G1202R, D1203N, F1245V, L1256F and G1269A.

5. The pharmaceutical composition for treating an ALK fusion gene-positive tumor according to any one of claims 1 to 3, wherein the ALK fusion gene-positive tumor has at least one ALK mutation of T1151K, C1159Y, I1171N, I1171T, I1171S, F1174I, F1174V, V1180L, L1196M, L1196Q, L1198F, D1203N, F1245V, L1256F or G1269A.

6. The pharmaceutical composition for treating an ALK fusion gene-positive tumor according to any one of claims 1 to 5, wherein the ALK fusion gene-positive tumor is a non-small cell lung cancer.

7. A pharmaceutical composition according to claim 6 for treating non-small cell lung cancer, for use as a primary therapy or a secondary therapy.

8. A composition for treating a tumor caused by activation of AXL, an NTRK fusion gene, an LTK fusion gene and/or a ROS1 fusion gene, containing gilteritinib as an active ingredient.

9. A method for examining whether gilteritinib exerts an effect on a patient with an ALK fusion-gene positive tumor, including: examining whether a kinase domain of ALK is a wild type or has at least one ALK mutation of T1151K, C1159Y, I1171N, I1171T, I1171S, F1174I, F1174V, V1180L, L1196M, L1196Q, L1198F, D1203N, F1245V, L1256F or G1269A; and determining that gilteritinib is effective when any one of the above present.

10. A method for examining whether gilteritinib exerts an effect on a patient with an ALK fusion-gene positive tumor, including examining whether G1202R or D1203 mutation is present; determining that gilteritinib does not exert an effect if G1202R is present alone, or determining that the effect of gilteritinib may be slightly attenuated if a compound mutant of D1203 mutation or G1202R mutation and another mutation is present.
